# EUROPEAN PATENT APPLICATION

(11) **EP 4 147 669 A1**
(43) Date of publication of application: **15.03.2023**
(21) Application number: 22191311.4
(22) Date of filing: 19.08.2022
(51) Int. Cl.: A61C 17/02, A61B 17/54, A61H 9/00, A61M 25/00, A61B 17/00

(54) **DERMAL INFUSION HANDPIECE WITH IMPROVED DELIVERY HEAD**

(30) Priority: 31.08.2021 US 202163238794 P; 11.11.2021 US 202163278128 P
(71) Applicant: TAV-TECH Ltd., 5630417 Yehud (IL)
(72) Inventor: CRANKO, Tim, 5591220 Ganei Tikva (IL); BOBOVA, Katia, Noa, 1825130 Afula (IL)
(74) Representative: Schulz Junghans Patentanwälte PartGmbB

(57) **Abstract**

A dermal treatment handpiece has a body with proximal and distal ends, a jet spray delivery head formed at the distal end of the body and connected to sources of a liquid suspension of a therapeutic agent and of a pressurized gas for a selectable combined supply thereof to the jet spray delivery head. The jet spray delivery head has a base formed at the distal end of the body, a localized environmental jet spray isolation chamber extending from the base to a distal exit port, a nozzle extending into the isolation chamber from the base, connected to a combined inflow of the liquid suspension and the pressurized gas, and one or more tubes mounted within the nozzle for receiving the combined inflow, each tube having a delivery tip extending into the isolation chamber for emitting a high pressure, high velocity jet spray along a delivery axis in response to receiving the combined inflow.

## Description

### FIELD OF INVENTION

The present disclosure generally relates to handheld devices for dermal administration of therapeutic agents.

### BACKGROUND

Handheld devices for dermal abrasion of exposed in vivo tissue are known in the art. One such device is described in the present applicant's International Publication No. WO 2005/065032, "A High Velocity Liquid-Gas Mist Tissue Abrasion Device", included herein by reference. This document also provides a general overview of dermal abrasion and dermal abrasion devices.

Disclosed in the above referenced document is a device for dermal abrasion employing a high-velocity liquid-gas jet spray, formed of droplets of a therapeutic agent in liquid suspension. The spray applied to the skin of a subject as a streaming mist. The mist-like properties of the spray may be advantageous for some purposes, whereas, for other purposes such as infusion, such properties, accompanied by a loss of speed, and a loss of force of the spray generally and of its component droplets in particular, the mist-like properties may render it less effective.

### DEFINITIONS

In the disclosure hereinbelow, the term "distal" refers to portions of the device furthest from a user operating the device, that is, closest to a jet spray delivery end of the device. The term "proximal" refers to portions of the device closest or most proximate to the user that is, the portion furthest from the jet spray delivery end of the device.

The term "tissue" as used herein can refer to either human or animal tissue.

### SUMMARY

The following embodiments and aspects thereof are described and illustrated in conjunction with systems, tools and methods which are meant to be exemplary and illustrative, not limiting in scope.

In addition to the exemplary aspects and embodiments described above, further aspects and embodiments will become apparent by reference to the figures and by study of the following detailed description.

There is thus provided, in accordance with an embodiment of the invention, a dermal treatment handpiece having a body having proximal and distal ends; and a jet spray delivery head formed at the distal end of the body, the delivery head being connected to a source of a liquid suspension of a therapeutic agent and to a source of a pressurized gas for a selectable combined supply of the liquid suspension and of the pressurized gas, to the jet spray delivery head, wherein the jet spray delivery head includes a base formed at the distal end of the body, a localized environmental jet spray isolation chamber extending from the base to a distal exit port, a nozzle extending from into the isolation chamber from the base, the nozzle being connected to a combined inflow of the liquid suspension and the pressurized gas, and one or more tubes mounted within the nozzle for receiving the combined inflow, each tube having a delivery tip extending into the isolation chamber for emitting a high pressure, high velocity jet spray along a delivery axis in response to receiving the combined inflow.

Additionally, the jet spray has a formational integrity prone to the influence of deleterious, localized air currents, and the isolation chamber is operative to reduce the influence of deleterious, localized air currents on the jet spray during its passage therethrough, thereby increasing the efficacy of the jet spray as it exits the isolation chamber through the distal exit port, into the environment.

Further, the isolation chamber is formed of a cylindrical wall extending axially away from the base, terminating in a circumferential edge defining the distal exit port.

Additionally, there is provided a single tube for providing a single jet spray along a delivery axis.

Alternatively, there are provided three tubes for providing a combined jet spray along a delivery axis.

### BRIEF DESCRIPTION OF THE FIGURES

Exemplary embodiments are illustrated in referenced figures. Dimensions of components and features shown in the figures are generally chosen for convenience and clarity of presentation and are not necessarily shown to scale. The figures are listed below.
Fig. 1 is a schematic illustration showing a handpiece of a dermal jet spray treatment system having a delivery head in accordance with an embodiment of the present invention;
Fig. 2 is an enlarged detailed view of a delivery head in accordance with an embodiment of the present invention, showing the atmospheric isolation chamber partially cut away;
Fig. 3 is a side view of the delivery head of Fig. 2, showing operation thereof;
Fig. 4 is a representation of the delivery head as in Fig. 3, showing impingement of the jet spray onto skin tissue during an infusion treatment, and the cavitation effect on the skin;
Fig. 5A is a detailed line drawing representation of the photograph of Fig. 5B;
Fig. 5B is a photograph showing use of a handpiece in an infusion treatment, having a PRIOR ART delivery head;
Fig. 6A is a detailed line drawing representation of the photograph of Fig. 6B;
Fig. 6B is a photograph showing use of a handpiece in an infusion treatment, having a delivery head of the present invention; and
Fig. 7 is a view similar to that of Fig. 3, but with a single tube.

### DETAILED DESCRIPTION

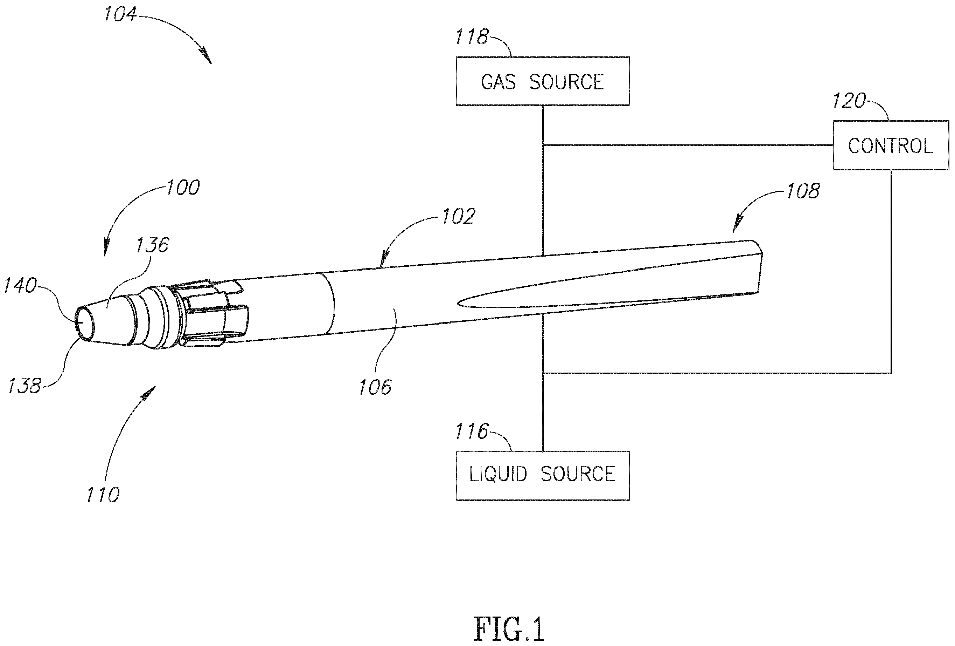

Referring now to Figs. 1-4, the present invention relates to an improved handpiece 102 of a dermal jet spray treatment system 104 (Fig. 1), such as manufactured and marketed by the current applicant, Tavtech Ltd. of Yehud, Israel, and as available at https://jetpeel.com, characterized by having an improved delivery head 100 with greater efficiency when compared to prior art devices. In a typical system, handpiece 102 has a body 106 with a proximal end 108 and a distal end 110 where the delivery head 100 is located.

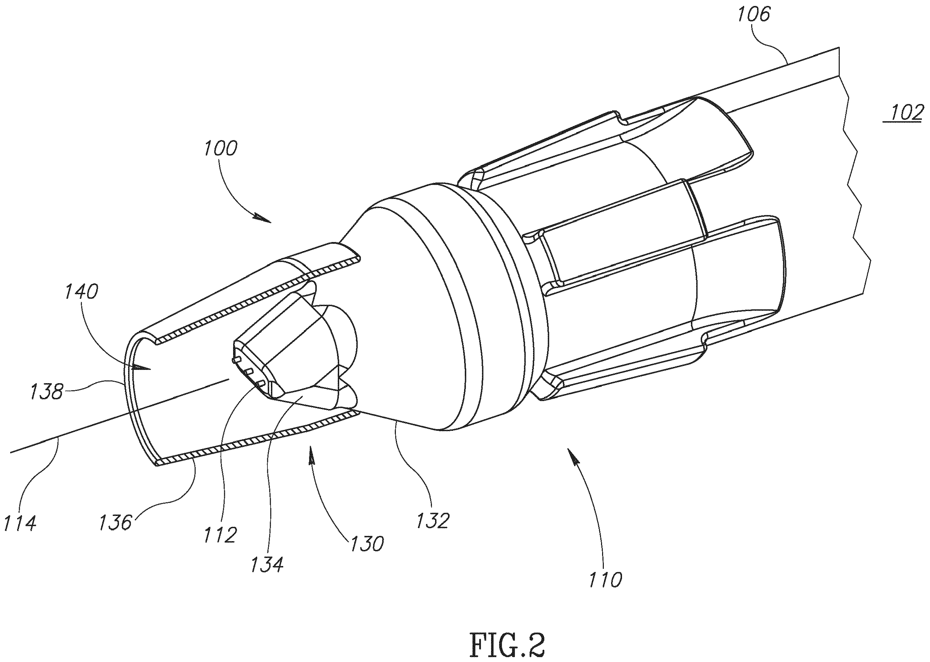

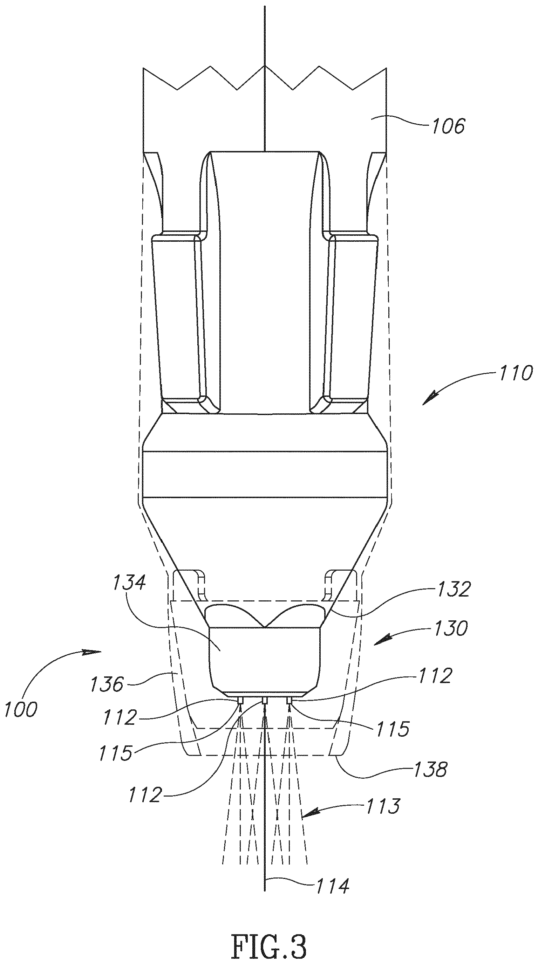

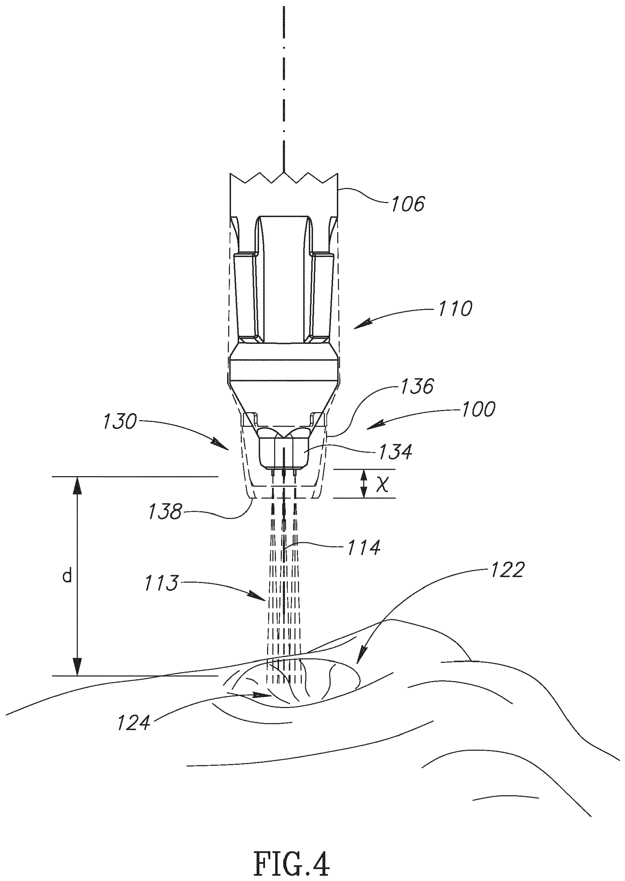

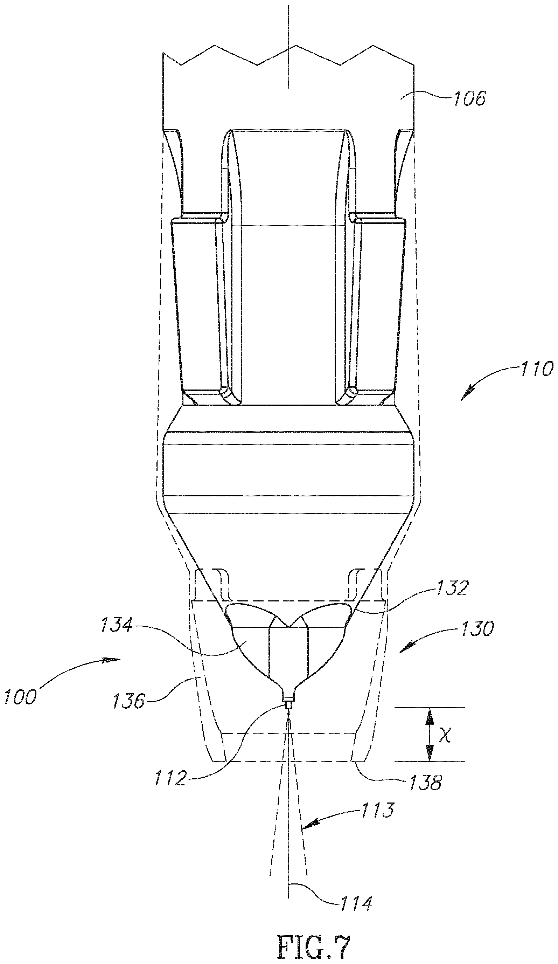

The delivery head 100 has one (Fig. 7) or more (Figs. 2-4) tubes 112 which are operative to deliver a high pressure, high velocity jet spray 113 (Figs. 3 and 4) along a delivery axis 114, produced, as indicated schematically in Fig. 1, by combining inflows of a liquid suspension of a therapeutic agent provided from a liquid source 116, and a pressurized gas provided from an appropriate gas source 118, operated by a suitable control 120. This method of producing a high pressure, high velocity jet spray is well known in the prior art, inter alia, in the above-referenced PCT publication no. WO 2005/065032 of the applicant, which describes a discharge of a liquid into a high velocity gas stream so as to form an accelerated mist or spray of the liquid. The precise manner in which this is facilitated for use in the present device can be in accordance with any prior art method, it is beyond the scope of the present invention and is thus not specifically described again herein.

The present description applies equally to the three-tube embodiment of Figs. 2-4 and to the single tube embodiment of Fig. 1, except for such differences arising directly from the different number of tubes in these different embodiments.

As seen in Fig. 4, an area of skin 122 of a subject to be treated is exposed to the jet spray 113 by holding the delivery head 100 close to the skin 122, at an exemplary distance ***d*** of 5-15 mm therefrom.

As known, the exposure of skin 122 to jet spray 113 is so as to provide a predefined therapy, for example, exfoliation or lymphatic massage. The present invention is particularly suited for infusion, as will be described below. Jet spray 113 provides a focused jet having predefined qualities, such as pressure, flow rate, spray pattern, droplet size, droplet speed, impact on skin, reach, and the like, so as to facilitate to enable trans-epidermal infusion, as mentioned.

The jet spray 113 is composed of high speed, high pressure microdroplets which bombard and thus impact the skin. The microdroplets possess kinetic energy which effectively causes them to act as solids when impacting the skin, and depending on the angle that the handpiece is held relative to the skin, to have an infusive effect thereon, so as to infuse the therapeutic agent bearing liquid into the skin. When the handpiece 102 is held perpendicular or nearly perpendicular to the skin, cavitation occurs, causing the formation of a recess or dimple 124 in the skin. This causes the skin in which recess 124 is formed to be stretched, causing the opening of micropores in the skin for infusion of the jet spray components, with gas and liquid vacuoles created in the skin tissue.

### Jet Spray Operating Conditions

This will be appreciated with reference to and in the context of a technical specification of a typical jet spray 113 emitted by delivery head 100:

| | |
|---|---|
| Jet spray pressure: | 85-95 psi |

Volumetric flowrate of spray: this can vary, depending on the number of tubes 112, and the diameter of the tubes which in the present, non-limiting example, are 32 gauge hypodermic tubes. A typical flow rate may be in the range 12-40 liter/min.

Liquid output: this too can vary in a manner similar to the flowrate, and is also affected by the viscosity of the liquid. By way of example, when using distilled water with the presently exemplified embodiment, a liquid output of 1.5 gram/min is obtained.

| | |
|---|---|
| Diameter of droplets in spray: | 7-17 µm. |
| Speed of droplets in spray: | 140 - 240 m/s |
| Impact Area on Skin when d = 5 mm: | 0.1 - 2.7 mm² |
| Impact Area on Skin when d = 10 mm: | 0.3 - 6 mm². |

It will be appreciated by persons skilled in the art that the formational integrity of the jet spray is of importance when providing an infusion treatment. This is due to the fact that while the jet spray 113 exits the delivery head 100 at high speed, the mass of the microdroplet spray is negligible, and is therefore highly susceptible to local atmospheric disturbances, particularly localized air currents such as may be caused by air conditioners, open windows, people walking close or even by the breath of the person holding and operating the handpiece.

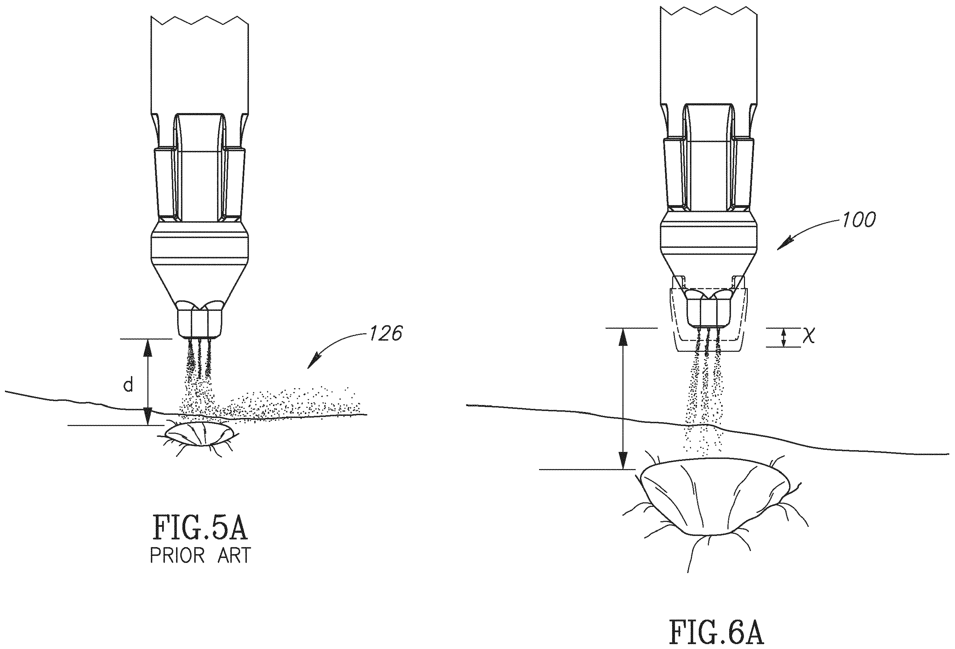

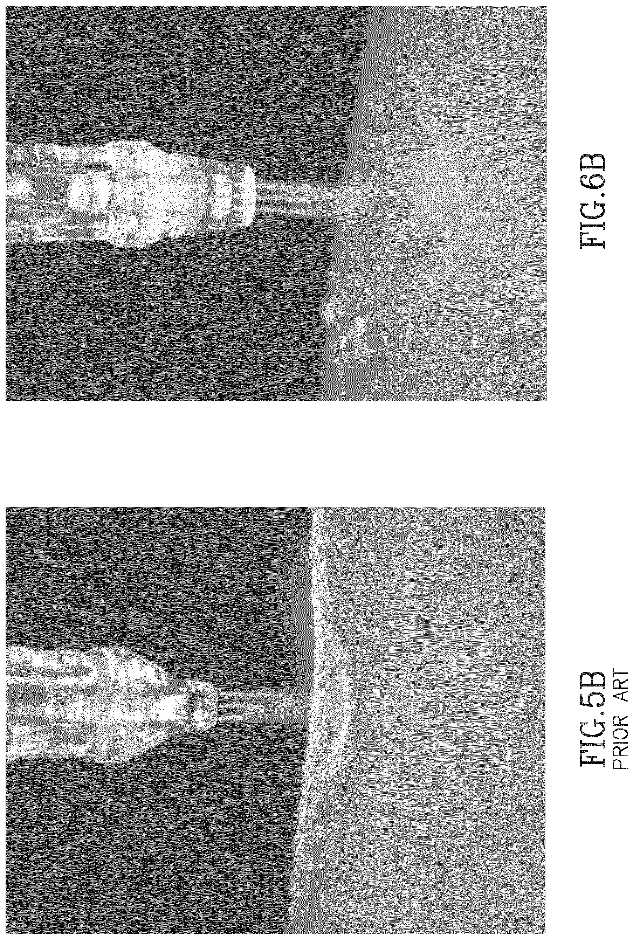

As will be appreciated from the comparison of an example of the invention with a corresponding example of the prior art below, in conjunction with Figs. 5A-6C, the formational integrity of the jet spray is an indication of its efficiency. This is due to the fact that while the jet spray contains significant kinetic energy, this energy is embodied in the aggregation of thousands of microdroplets, each of which has negligible mass. Therefore, the slightest disturbance to the formational integrity of the spray, as by localized air flow, causes a diversion of significant numbers of the microdroplets, thus preventing the kinetic energy from meeting its target, namely, the skin 122 to be treated. This is clearly illustrated in and described on conjunction with the PRIOR ART example of Figs. 5A-5B.

Referring now briefly to Figs. 5A-5B, the illustrated PRIOR ART delivery head, exemplified as the "Triple" handpiece, as seen at https://jetpeel.com/jetpeel/jetpeel-handpieces/, has three tubes 112. The specific operating conditions used in the pictured example were as follows:

| | |
|---|---|
| Jet Spray Pressure: | 95 psi |
| Distance ***d*** from the skin: | 15 mm |

Note that the distance d as illustrated in Fig. 5A refers to the distance between the tube tips and the skin in its pre-cavitated state.

In addition to the formation of a dimple due to the above-described cavitation, a mist cloud 126 is seen to form in the vicinity of the spray, caused by deleterious, localized atmospheric disturbances, such as described above. This reduces the effectiveness of the infusion treatment that may be provided with a prior art handpiece as reflected in the shallowness of the dimple. This is described in greater detail below.

Referring now again to Figs. 2-4, it is seen that the delivery end 110 of the body 106 has formed thereat a localized atmospheric isolation chamber 130 extending distally from a base 132 to a distal exit port 134. Mounted onto and extending away from base 132 is a nozzle 134 which, in turn, has mounted therein one or more tubes 112. Each tube has a jet spray delivery tip 115 which protrudes into the isolation chamber 130. In the present example, three tubes 112 are provided, as seen, although 1, 2 or any other predetermined number of tubes 112 may be provided. Examples of prior art handpieces with varying numbers of tubes may be found, for example, at https://jetpeel.com/jetpeel/jetpeel-handpieces, including the single tube "Magic", "Single" or "Prestige Plus" handpieces, or the "Double" handpiece with two tubes, any of which may be employed with the delivery head of the present invention.

The isolation chamber 130 is formed of a side wall 136, typically generally cylindrical or frustoconical in shape, which extends axially away from the base 132 so as to surround and extend beyond the delivery tips 115 (Fig. 4) of the tubes 112. Wall 136 terminates in a circumferential edge or lip 138 which defines a distal exit port 140 through which jet spray 113 passes.

It has been found by the inventors that in order to sufficiently minimize localized atmospheric disturbances to the formational integrity of the spray, it is sufficient to prevent such disturbances to the emergent portion only, of the spray as it leaves tube tips 115, indicated in Fig. 4 by dimension x, which is typically in the range 1.0 - 2.0 mm. This compares with the typical distance of the tube tips 115 from the skin, of 5-15mm. This protection is provided by a corresponding protrusion of lip 138 which protrudes by at least the same dimension ***x*** beyond the delivery ends or tips 115 of the tubes, wherein ***x*** is typically in the range 1.0 - 2.0 mm.

In the example pictured in Fig. 6A, ***x*** = 2.0 mm, approximately.

Referring now specifically to Figs. 6A-6B, there are seen both a photograph and a line drawing representation of a demonstration of use of the system 104, in which a handpiece 102 is used, identical to the prior art handpiece of Figs. 5A-5B, but modified to include isolation chamber 130. The operating conditions of the demonstration depicted in Figs. 6A-6B are similar to those of the demonstration of the prior art device, as described above in conjunction with Figs. 5A-5B.

The cavitation effectiveness of the delivery head 100 of the invention (Figs. 6A-6B) when compared to that of the prior art (Figs. 5A-5B) many be adjudged by comparing the width and depth of the resulting dimple 124. As may be seen, use of the delivery head of the invention results in more focused, significantly deeper dimple, corresponding to the protection provided to the jet spray 113 when initially exiting tips 115 of tubes 112. As discussed above, isolation of the jet spray, even for a portion of its travel after leaving tips 115, prevents partial dispersal as indicated by mist 126 (Fig. 5A), thus preserving the formational integrity and force of the spray, enabling deeper cavitation, increased stretching of the micropores and more effective infusion.

It will further be appreciated, that a further advantage of provision of the isolation chamber is to protect the delivery tips 115 of the tubes 112 from mechanical damage, whether by inadvertently touching the skin of a person being treated, or otherwise being knocked when not in use.

As used herein the term "configuring" and/or 'adapting' for an objective, or a variation thereof, implies using materials and/or components in a manner designed for and/or implemented and/or operable or operative to achieve the objective. Unless otherwise specified, the terms 'about' and/or 'close' with respect to a magnitude or a numerical value implies within an inclusive range of -10% to +10% of the respective magnitude or value. Unless otherwise specified, the terms 'about' and/or 'close' with respect to a dimension or extent, such as length, implies within an inclusive range of - 10% to +10% of the respective dimension or extent. Unless otherwise specified, the terms 'about' or 'close' imply at or in a region of, or close to a location or a part of an object relative to other parts or regions of the object.

When a range of values is recited, it is merely for convenience or brevity and includes all the possible sub ranges as well as individual numerical values within and about the boundary of that range. Any numeric value, unless otherwise specified, includes also practical close values enabling an embodiment or a method, and integral values do not exclude fractional values. A sub range of values and practical close values should be considered as specifically disclosed values. As used herein, ellipsis (...) between two entities or values denotes an inclusive range of entities or values, respectively. For example, A...Z implies all the letters from A to Z, inclusively.

The terminology used herein should not be understood as limiting, unless otherwise specified, and is for the purpose of describing particular embodiments only and is not intended to be limiting of the disclosed subject matter. While certain embodiments of the disclosed subject matter have been illustrated and described, it will be clear that the disclosure is not limited to the embodiments described herein. Numerous modifications, changes, variations, substitutions and equivalents are not precluded. Terms in the claims that follow should be interpreted, without limiting, as characterized or described in the specification.

The descriptions of the various embodiments of the present invention have been presented for purposes of illustration, but are not intended to be exhaustive or limited to the embodiments disclosed. Many modifications and variations will be apparent to those of ordinary skill in the art without departing from the scope and spirit of the described embodiments. The terminology used herein was chosen to best explain the principles of the embodiments, the practical application or technical improvement over technologies found in the marketplace, or to enable others of ordinary skill in the art to understand the embodiments disclosed herein.

## Claims

1. A dermal treatment handpiece which comprises:
a body having proximal and distal ends;
a jet spray delivery head formed at said distal end of said body, said delivery head being connected to a source of a liquid suspension of a therapeutic agent and to a source of a pressurized gas for a selectable combined supply of the liquid suspension and of the pressurized gas, to said jet spray delivery head;
wherein said jet spray delivery head comprises:
a base formed at said distal end of said body;
a localized environmental jet spray isolation chamber extending from said base to a distal exit port;
a nozzle extending from into said isolation chamber from said base, said nozzle being connected to a combined inflow of the liquid suspension and the pressurized gas; and
at least one tube mounted within said nozzle for receiving the combined inflow, each said at least one tube having a delivery tip extending into said isolation chamber for emitting a high pressure, high velocity jet spray along a delivery axis in response to receiving the combined inflow.

2. A handpiece according to claim 1, wherein the jet spray has a formational integrity prone to the influence of deleterious, localized air currents, and wherein said isolation chamber is operative to reduce the influence of deleterious, localized air currents on the jet spray during its passage therethrough, thereby increasing the efficacy of the jet spray as it exits said isolation chamber through said distal exit port, into the environment.

3. A handpiece according to claim 2, wherein said isolation chamber comprises a cylindrical wall extending axially away from said base, said cylindrical wall terminating in a circumferential edge defining said distal exit port.

4. A handpiece according to claim 1, wherein said at least one tube comprises a single tube for providing a single jet spray along a delivery axis.

5. A handpiece according to claim 1, wherein said at least one tube comprises three tubes for providing a combined jet spray along a delivery axis.
